# EUROPÄISCHE PATENTSCHRIFT

(11) **EP 4 231 009 B1**
(45) Veröffentlichungstag und Bekanntmachung des Hinweises auf die Patenterteilung: **29.01.2025**
(21) Anmeldenummer: 22157753.9
(22) Anmeldetag: 21.02.2022
(51) Int. Cl.: G01N 30/32, G01N 33/00

(54) **GASANALYSEVORRICHTUNG MIT PNEUMATIKMODUL UND HERSTELLUNGSVERFAHREN UND COMPUTERPROGRAMMPRODUKT DAFÜR**
PNEUMATIC MODULE FOR GAS ANALYSIS DEVICE, AND MANUFACTURING METHOD AND COMPUTER PROGRAM PRODUCT THEREFOR
MODULE PNEUMATIQUE POUR DISPOSITIF D'ANALYSE DE GAZ, ET PROCÉDÉ DE FABRICATION ET PRODUIT-PROGRAMME INFORMATIQUE POUR CELUI-CI

(43) Veröffentlichungstag der Anmeldung: 23.08.2023
(73) Patentinhaber: Siemens Aktiengesellschaft, 80333 München (DE)
(72) Erfinder: RICHTER, Josef, 76185 Karlsruhe (DE); STRAUCH, Piotr, 76761 Rülzheim (DE)
(74) Vertreter: Siemens Patent Attorneys

(56) Entgegenhaltungen:
- DE-A1- 1 498 517
- DE-A1- 19 814 650
- DE-A1- 4 113 695
- US-A- 5 010 776
- US-A1- 2017 286 572
- US-A1- 2020 326 320

## Beschreibung

Die Erfindung betrifft ein Pneumatikmodul für eine Gasanalysevorrichtung und eine Gasanalysevorrichtung, die mit einem solchen Pneumatikmodul ausgestattet ist. Die Erfindung betrifft ebenso ein Herstellungsverfahren für ein derartiges Pneumatikmodul. Ferner betrifft die Erfindung ein Computerprogrammprodukt zum Simulieren eines Betriebsverhaltens eines entsprechenden Pneumatikmoduls.

Aus der Patentschrift US 10,192,723 B2 ist eine Vorrichtung zur massenspektrometrischen Analyse von Stoffen bekannt, in der eine Quarzkapillare angeordnet ist, über die eine Stoffprobe zu einer Düse geführt wird. Benachbart zur Düse ist ein Massenspektrometer angeordnet.

Die Druckschrift US 2002/0180109 A1 offenbart eine Drossel, die durch eine Klemme aus einem Rohling geformt wird. Beim Umformen mit der Klemme ist ein Dorn in den Rohling eingeführt.

Aus der Bedienungsanleitung mit dem Titel "Prozess-Gaschromatograph MicroSAM" der Siemens AG, Ausgabe 03/2012, ist eine Gasanalysevorrichtung bekannt, in der Lastströmeinsätze angeordnet sind.

Die Offenlegungsschrift DE 14 98 517 A1 zeigt ein Gaseinlass-System für Massenspektrometer, das als Versorgungsleitung eine Kapillare enthält, die an eine Gasquelle angeschlossen ist. Dieses umfasst einen röhrenförmigen Körper, in dem ein röhrenförmiger Ventilkolben aufgenommen ist, in der wiederum eine Kapillare angeordnet ist.

Aus der Patentanmeldung DE 198 14 650 A1 ist ein Verfahren zur Herstellung einer Drosselstelle bekannt, die einen Schlauch aufweist, an dem eine Sicherungsklammer angebracht ist. Zur Herstellung der Drosselstelle wird der Schlauch unter Überdruck gesetzt, um diesen außerhalb der Sicherungsklammer aufzuweiten. Der so umgeformte Schlauch ist als Drossel für eine Gasströmung geeignet.

Ferner geht aus der Druckschrift DE 41 13 695 A1 ein kontinuierlich betriebener Gasanalysator hervor, der einen Detektor aufweist, dem eine erste Drossel vorgeschaltet ist. Ebenso weist der Gasanalysator eine zweite Drossel auf, die als Bypass zum Detektor mit der ersten Drossel angeordnet ist. Weiter weist der Gasanalysator eine dritte Drossel auf, die einem gemeinsamen Gaseintritt vorgeschaltet ist.

Bei Gasanalysevorrichtungen besteht die Zielsetzung, deren Messgenauigkeit und Robustheit gegenüber den vorliegenden Betriebsbedingungen zu erhöhen. Weiter wird angestrebt, Gasanalysevorrichtungen kompakt auszubilden und kosteneffizient herzustellen. Ebenso besteht der Wunsch nach einer erhöhten Reparaturfreundlichkeit. Der Erfindung liegt die Aufgabenstellung zugrunde, eine Möglichkeit bereitzustellen, die in zumindest einem der skizzierten Aspekte eine Verbesserung bietet.

Die zugrundeliegende Aufgabe durch eine erfindungsgemäße Gasanalysevorrichtung gelöst. Die Gasanalysevorrichtung umfasst einen Leitungsblock, der eine Mehrzahl an Kanälen aufweist. Die Mehrzahl an Kanälen sind als Ausnehmungen im Leitungsblock ausgebildet. Zumindest zwei Kanäle sind über ein austauschbares Pneumatikmodul miteinander verbunden. Einer der Kanäle kann beispielsweise als Zufuhrkanal für eine Fluidströmung, der andere Kanal als Ablaufkanal für die Fluidströmung ausgebildet sein. Durch die Verbindung über das austauschbare Pneumatikmodul tritt eine Fluidströmung aus einem der Kanäle in den anderen Kanal. Durch das austauschbare Pneumatikmodul wird auf die Fluidströmung strömungsmechanisch eingewirkt, beispielsweise in Form einer Drosselung, also durch Hervorrufen eines Druckverlusts.

Erfindungsgemäß ist das austauschbare Pneumatikmodul dazu ausgebildet, eine Fluidströmung in einer Gasanalysevorrichtung einzustellen. Das Einstellen der Fluidströmung erfolgt, indem mittels des Pneumatikmoduls ein definierter Strömungswiderstand bereitgestellt wird, so dass bei entsprechender Stärke der Fluidströmung in dieser ein Absolutdruckverlust eintritt. Die Fluidströmung kann beispielsweise eine Strömung einer Stoffprobe, eines Trägergases, oder eines Gemischs daraus sein. Das Pneumatikmodul weist eine Trägerhülse auf, in der ein Strömungsmodul aufgenommen ist. Das Strömungsmodul wird im Betrieb des Pneumatikmoduls von der Fluidströmung durchströmt und ist dazu ausgebildet, auf diese einzuwirken, beispielsweise für die Fluidströmung einen Strömungswiderstand darzustellen. Erfindungsgemäß ist das Strömungsmodul an seinem ersten Ende, also im Bereich seines ersten Endes, mit der Trägerhülse verbunden. Durch die Verbindung am ersten Ende ist das Strömungsmodul ist dieses im Wesentlichen durchgängig von der Trägerhülse umgeben. Zwischen der Trägerhülse und dem Strömungsmodul ist ein lichter umlaufender Zwischenraum ausgebildet. Das Strömungsmodul wird von der Trägerhülse gegen mechanische und thermische Einwirkungen aus einer Umgebung abgeschirmt. Je geringer eine Wandstärke, ein Innendurchmesser und/oder Außendurchmesser des Strömungsmoduls ist, umso empfindlicher ist es gegen derartige Einwirkungen. Dadurch ist die Wirkung des Strömungsmoduls auf die Fluidströmung beeinträchtigbar, was wiederum die erreichbare Messgenauigkeit reduziert. Durch die Trägerhülse werden mechanische Einwirkungen auf das Strömungsmodul abgehalten. Die Verbindung zwischen der Trägerhülse und dem Strömungsmodul an dessen erstem Ende ist kompakt ausbildbar, so dass eine Kontaktfläche, die eine Wärmeleitung von der Trägerhülse auf das Strömungsmodul oder umgekehrt erlaubt, minimiert ist.

Dadurch wird die thermische Abschirmung des Strömungsmoduls durch die Trägerhülse gewährleistet und gleichzeitig eine stabile Befestigung des Strömungsmoduls bereitgestellt. Dies ermöglicht unter anderem eine gesteigerte Betriebstemperatur des Pneumatikmoduls. Infolge der thermischen Abschirmung durch die Trägerhülse dient die Verbindung zwischen der Trägerhülse und dem Strömungsmodul an dessen erstem Ende einer Steigerung der Betriebstemperatur des Pneumatikmoduls. Die Verbindung zwischen der Trägerhülse und dem Strömungsmodul an dessen ersten Ende dient ebenso einer Steigerung der Messgenauigkeit, die mit der Gasanalysevorrichtung erzielbar ist, in der ein solches Pneumatikmodul eingesetzt wird.

Erfindungsgemäß ist die Trägerhülse im Bereich des ersten Endes des Strömungsmoduls zu einer Montage an der Gasanalysevorrichtung ausgebildet. Die Trägerhülse kann dazu an ihrer Außenfläche beispielsweise mit einem Gewinde, einem Haltevorsprung, einem Absatz und/oder einem Dichtmittel wie einem O-Ring versehen sein. Weiter erfindungsgemäß ist beim beanspruchten Pneumatikmodul die Trägerhülse in einem Bereich des zweiten Endes des Strömungsmoduls verschlossen ausgebildet. Eine am zweiten Ende des Strömungsmoduls austretende Fluidströmung wird so an einem Austreten in die Umgebung gehindert und ist durch die Trägerhülse im lichten umlaufenden Zwischenraum in einen Bereich des ersten Endes des Strömungsmoduls zurückführbar. Insgesamt wird so eine erhöhte Dichtwirkung erreicht.

Das Pneumatikmodul ist dadurch in einfacher Weise montierbar und austauschbar. Beispielsweise kann die Trägerhülse zumindest abschnittsweise kantig ausgebildet sein, die ein Festschrauben oder Lösen mittels eines Werkzeugs wie einem Steckschlüssel erlaubt. Alternativ oder ergänzend kann an eine Stirnfläche an einem freien Ende des Pneumatikmoduls eine Ausnehmung zum Ansetzen eines Werkzeugs aufweisen, beispielsweise eine Innensechskantbohrung. Weiter kann die Gasanalysevorrichtung ein Heizelement und/oder Kühlelement aufweisen, das benachbart zum Pneumatikmodul angeordnet ist. Unter einer benachbarten Anordnung ist zu verstehen, dass eine vom Heizelement abgegebene Wärme bzw. vom Kühlelement aufgenommene Wärme in erfassbarer Form auf das Pneumatikmodul einwirkt. Das beanspruchte Pneumatikmodul bietet für das darin angeordnete Strömungsmodul eine hinreichende thermische Abschirmung, so dass trotz der Positionierung des Pneumatikmoduls benachbart zum Heizelement bzw. Kühlelement weiterhin eine erhöhte Messgenauigkeit mit der Gasanalysevorrichtung erzielbar ist. Die Gasanalysevorrichtung kann als Gasanalysator oder als Gaschromatograph ausgebildet sein. Insgesamt wird mittels des beanspruchten Pneumatikmoduls die mit der Gasanalysevorrichtung gesteigert. Ferner ist das Pneumatikmodul schnell und einfach austauschbar. Insbesondere in Verbindung mit dem Leitungsblock sind bei der Gasanalysevorrichtung ein besonders robuster Betrieb und eine erhöhte Reparaturfreundlichkeit möglich.

In einer Ausführungsform des beanspruchten Pneumatikmoduls ist das Strömungsmodul an seinem ersten Ende mit der Trägerhülse unlösbar verbunden. Unter einer unlösbaren Verbindung ist eine nur zerstörungsbehaftet lösbare Verbindung zu verstehen. Insbesondere kann das Strömungsmodul mit der Trägerhülse stoffschlüssig verbunden sein, beispielsweise über eine Schweißverbindung, eine Punktschweißverbindung, Laserschweißverbindung, Elektronenstrahlschweißverbindung, eine Lötverbindung, eine Hartlötverbindung oder eine Klebverbindung. Derartige unlösbare Verbindungen sind maschinell mit erhöhter Präzision prozesssicher herstellbar. Eine derartige unlösbare Verbindung bietet eine gesteigerte Dichtwirkung. Die unlösbare Verbindung kann im Bereich des ersten Endes zylindermantelflächenförmig ausgebildet sein. Je länger diese ausgebildet ist, umso stärker ist die erzielte Dichtwirkung. Weiter ist ein Wärmeeintrag in das Strömungsmodul bei der Herstellung bei derartigen Verbindungen reduzierbar. Je geringer der Wärmeeintrag in das Strömungsmodul ist, umso geringer ist dessen Verzug. Das beanspruchte Pneumatikmodul ist in einem erhöhten Grad maschinell fertigbar, wodurch dessen Herstellung schnell, präzise und kosteneffizient durchführbar ist. Ebenso können dadurch filigranere, also dünnwandigere, Strömungsmodule und/oder Trägerhülsen verwendet werden. Dies wiederum erlaubt eine weitergehende Miniaturisierung des Pneumatikmoduls und einer damit ausgestatteten Gasanalysevorrichtung. Alternativ kann zwischen der Trägerhülse und dem Strömungsmodul im Bereich des ersten Endes eine lösbare Verbindung ausgebildet sein. Eine solche lösbare Verbindung kann beispielsweise als Schraubverbindung oder Bajonettverbindung ausgebildet sein.

Darüber hinaus kann im beanspruchten Pneumatikmodul ein zweites Ende des Strömungsmoduls in einen Innenraum der Trägerhülse münden. Das Strömungsmodul kann im Wesentlichen rohrförmig ausgebildet sein, so dass eine über das erste Ende eintretende Fluidströmung am zweiten Ende in den Innenraum der Trägerhülse austreten kann. Die Trägerhülse erstreckt sich entlang ihrer Hauptachse über das zweite Ende des Strömungsmoduls hinaus. Dadurch ist das zweite Ende des Strömungsmoduls gegenüber der Umgebung abgeschirmt. Das zweite Ende ist dadurch bei der Herstellung frei von unmittelbaren Wärmeeinträgen, wodurch im Bereich des zweiten Endes einem Verzug durch thermische Einwirkung vorgebeugt wird. Das Strömungsmodul ist daher im Bereich seines zweiten Endes filigran, also beispielsweise mit verringerter Wandstärke, ausbildbar.

Weiter kann die Trägerhülse dazu ausgebildet sein, dass im montierten Zustand in der Gasanalysevorrichtung die Fluidströmung am ersten Ende des Strömungsmoduls in das Pneumatikmodul eintritt. Die Trägerhülse kann im Bereich des ersten Endes des Strömungsmoduls als ein standardisierter Anschluss ausgebildet sein. Folglich sind unterschiedliche Strömungsmodule in gleichartige Trägerhülsen einsetzbar. Dies erlaubt bei der Herstellung des beanspruchten Pneumatikmoduls für die Trägerhülse die Verwendung von Gleichteilen. Anschlüsse für das beanspruchte Pneumatikmodul können folglich in der zugehörigen Gasanalysevorrichtung baugleich ausgebildet sein. Der Herstellungsaufwand für das beanspruchte Pneumatikmodul und die entsprechende Gasanalysevorrichtung wird dadurch reduziert. Ebenso wird ein Kontakt, insbesondere ein Handkontakt, mit dem Strömungsmodul minimiert. Das Eindringen von Verunreinigungen wie Fette oder Öle, die in einem Messbetrieb der Gasanalysevorrichtung störend wirken, wird verhindert. Weiter kann das Pneumatikmodul eine maximale Betriebstemperatur, also eine maximale Temperatur der im Betrieb durchströmenden Fluidströmung, aufweisen, die im Wesentlichen einer maximalen Betriebstemperatur zumindest eines der Dichtmittel entspricht. Das beanspruchte Pneumatikmodul bietet eine hinreichende thermische Abschirmung des Strömungsmoduls, dass die Dichtmittel für das Pneumatikmodul thermisch dimensionierend sind. Insbesondere kann die maximale Betriebstemperatur des Pneumatikmoduls bis zu 40°C, bevorzugt bis zu 15°C, besonders bevorzugt bis zu 10°C unter der maximalen Betriebstemperatur eines der Dichtmittel liegen.

In einer weiteren Ausführungsform des beanspruchten Pneumatikmoduls kann die Trägerhülse einstückig oder mehrstückig ausgebildet sein. Durch eine einstückige Trägerhülse wird die Anzahl an Fertigungsschritten reduziert und die Anzahl an Dichtstellen minimiert. Bei einer mehrstückigen Trägerhülse kann beispielsweise ein erster Abschnitt, in dem das erste Ende des Strömungsmoduls mit der Trägerhülse verbunden ist, mit einer engeren Toleranz gefertigt sein als ein daran angrenzender zweiter Abschnitt, der das Strömungsmodul im Wesentlichen nur durch den lichten Zwischenraum beabstandet umgibt. Alternativ oder ergänzend können der erste und zweite Abschnitt der Trägerhülse aus unterschiedlichen Werkstoffen hergestellt sein, was eine beanspruchungsgerechte Werkstoffauswahl erlaubt. Je nach der erforderlichen Fertigungstoleranz können der erste bzw. zweite Abschnitt der Trägerhülse auf unterschiedliche Weise hergestellt werden. Hierdurch wird eine gesteigerte Kosteneffizienz bei der Herstellung des beanspruchten Pneumatikmoduls gewährleistet.

Die Trägerhülse kann ferner mit einer Austrittöffnung versehen sein, insbesondere im Bereich des ersten Endes des Strömungsmoduls. Die Austrittsöffnung an der Trägerhülse kann beispielsweise als Austrittsbohrung ausgebildet sein, beispielsweise, bezogen auf die Hauptachse der Trägerhülse, als Radialbohrung. Das beanspruchte Pneumatikmodul ist insgesamt einfach und robust in der Gasanalysevorrichtung montierbar. Insbesondere wird durch die im Bereich des zweiten Endes verschlossene Trägerhülse eine maschinelle Handhabung bei einer Montage, beispielsweise durch einen Roboter, vereinfacht. Das beanspruchte Pneumatikmodul erlaubt daher bei der Herstellung von entsprechenden Gasanalysevorrichtungen einen gesteigerten Automatisierungsgrad. Ferner ist dadurch eine weitere Miniaturisierung des beanspruchten Pneumatikmoduls möglich.

Ferner kann die Trägerhülse im Bereich des zweiten Endes des Strömungsmoduls durch einen unlösbar befestigten Deckel verschlossen ausgebildet sein. Unter einer unlösbaren Befestigung ist hierbei eine höchstens zerstörungsbehaftet lösbare Verbindung zu verstehen. Der Deckel kann beispielsweise stoffschlüssig, also durch eine Schweißverbindung, eine Punktschweißverbindung, Laserschweißverbindung, Elektronenstrahlschweißverbindung, eine Lötverbindung, eine Hartlötverbindung oder eine Klebverbindung mit der Trägerhülse verbunden sein. Dementsprechend kann die Trägerhülse im Bereich des zweiten Endes des Strömungsmoduls entlang ihrer Hauptachse eine Montageöffnung aufweisen. Die Montageöffnung ist zu einem Einführen des Strömungsmoduls ausgebildet und entsprechend dimensioniert. Insbesondere kann die Montageöffnung derart bemessen sein, dass das Strömungsmodul durch sie einführbar und während eines Verbindens mit der Trägerhülse im Bereich des ersten Endes des Strömungsmoduls haltbar ist. Dies erlaubt eine weiter automatisierte Herstellung des Pneumatikmoduls. Der Deckel ist an der Montageöffnung in einem weiteren Schritt separat mit der Trägerhülse verbindbar. Der Deckel ist in einfacher Weise zuverlässig dicht mit der Trägerhülse verbindbar. Durch die so erzielte stärkere Automatisierung wird die Herstellung des beanspruchten Pneumatikmoduls weiter beschleunigt. Gleichermaßen wird so die Verwendung von filigraneren Strömungsmodulen ermöglicht, indem gegenüber bekannten Lösungen die von Personen durchgeführten Handhabungen des Strömungsmoduls minimiert werden.

In einer weiteren Ausführungsform des beanspruchten Pneumatikmoduls sind das Strömungsmodul und/oder die Trägerhülse zumindest teilweise aus einem metallischen Werkstoff hergestellt. Insbesondere können die Trägerhülse und das Strömungsmodul aus metallischen Werkstoffen hergestellt sein, die vorteilhafte Eigenschaften in puncto gegenseitige Stoffschlüssigkeit aufweisen, beispielsweise Schweißbarkeit. Metallische Werkstoffe bieten eine hinreichende Stabilität, die eine maschinelle Montage des Pneumatikmoduls erlauben. Des Weiteren können das Strömungsmodul und die Trägerhülse aus Werkstoffen hergestellt sein, die in stoffschlüssiger Verbindung einen gesteigerten Wärmeleitungswiderstand aufweisen. Dadurch ist ein Wärmeeintrag aus der Trägerhülse in das Strömungsmodul weiter reduzierbar. Des Weiteren können das Strömungsmoduls und/oder die Trägerhülse mit einer inertisierenden Beschichtung versehen sein. Mittels der inerstisierenden Beschichtung werden chemische Reaktionen zwischen den metallischen Oberflächen und dem durchströmenden Fluid minimiert, und so Störeinflüsse im Messbetrieb verringert. Alternativ oder ergänzend kann die Trägerhülse auch aus einem Kunststoff, beispielsweise einem Faserverbundwerkstoff, hergestellt sein, der erhöhte Widerstandsfähigkeit gegen Wasserstoffversprödung bietet. Dementsprechend ist das beanspruchte Pneumatikmodul beständig für einen Dauerbetrieb mit Wasserstoff oder einem wasserstoffhaltigen Gasgemisch als Fluid.

Darüber hinaus kann das Strömungsmodul einen minimalen Innendurchmesser von 1 µm bis zu 2 mm aufweisen und/oder eine Wandstärke von bis zu 5 mm. Unter dem minimalen Innendurchmesser ist insbesondere ein minimaler Innendurchmesser im zweiten Abschnitt der Trägerhülse zu verstehen, durch den sich das Strömungsmodul erstreckt. Für derartige Abmessungen hinreichend präzise Herstellungsverfahren sind in einfacher Weise verfügbar. Das Strömungsmodul ist im Wesentlichen dahingehend dimensioniert, dass für den jeweiligen Anwendungsfalls erforderliche aerodynamische Ähnlichkeitszahlen, beispielsweise die Reynolds-Zahl oder die Rohrreibungszahl, erreicht wird. Auf die Fluidströmung kann somit auch bei entsprechenden Abmessungen des Strömungsmoduls gezielt eingewirkt werden. Gleichzeitig wird die Verwendung derart kompakter bzw. filigraner Strömungsmodul durch das beanspruchte Pneumatikmodul praxistauglich, da die Trägerhülse ausreichenden Schutz vor Einwirkungen aus der Umgebung bietet und eine einfache Handhabung ermöglicht.

In einer weiteren Ausführungsform des beanspruchten Pneumatikmoduls kann das Strömungsmodul als eine Quetschdrossel ausgebildet sein. Die Quetschdrossel ist im Wesentlichen als Röhrchen ausgebildet, das in einem mittleren Bereich durch plastisches Verformen einen verkleinerten Querschnitt aufweist. Die Quetschdrossel ist dazu ausgebildet, auf die Fluidströmung als Strömungswiderstand einzuwirken, also einen definierten Druckverlust in der Fluidströmung hervorzurufen. Quetschdrosseln sind anfällig gegen Wärmeausdehnung, und mechanische Verformung, so dass die Wirkung als Strömungswiderstand bei Wärmeeintrag oder Verbiegung beeinflusst wird. Das beanspruchte Pneumatikmodul bietet einen gesteigerten Schutz gegen Wärmeeintrag aus der Umgebung, mechanische Verformung und gewährleistet einen zuverlässigen und präzisen Betrieb und erhöhte Wartungsfreundlichkeit der Quetschdrossel. Folglich ist die Fluidströmung mittels des beanspruchten Pneumatikmoduls besonders präzise einstellbar und reproduzierbar. Dies wiederum erlaubt einen Betrieb der Gasanalysevorrichtung mit einer gesteigerten Zuverlässigkeit und Messgenauigkeit. Weiter kann das Strömungsmodul als Blende ausgebildet sein.

Die eingangs beschriebene Aufgabenstellung wird ebenso durch ein erfindungsgemäßes Verfahren zum Herstellen eines Pneumatikmoduls gelöst, das zur Verwendung in einer Gasanalysevorrichtung geeignet ausgebildet ist. Die Gasanalysevorrichtung kann beispielsweise als Gasanalysator, insbesondere als sogenannter continuous gas analyzer, kurz CGA, oder als Gaschromatograph ausgebildet sein. Das Verfahren umfasst einen ersten Schritt, in dem eine Trägerhülse und ein Strömungsmodul bereitgestellt werden, aus denen das Pneumatikmodul herzustellen ist. Das Verfahren umfasst weiter einen zweiten Schritt, in dem das Strömungsmodul in einen Innenraum der Trägerhülse eingeführt wird. Das Einführen umfasst, dass das Strömungsmodul in einer vorgesehenen Position gehalten wird, in der es zu befestigen ist. Hierbei wird ein erstes Ende des Strömungsmoduls unmittelbar benachbart zur Trägerhülse platziert. Des Weiteren weist das Verfahren einen dritten Schritt auf, in dem eine unlösbare Verbindung zwischen der Trägerhülse und dem Strömungsmodul im Bereich des ersten Endes des Strömungsmoduls hergestellt wird, so dass diese das herzustellende Pneumatikmodul bilden. Durch den dritten Schritt sind die Trägerhülse und das Strömungsmodul nur noch zerstörungsbehaftet voneinander trennbar. Die unlösbare Verbindung kann beispielsweise als stoffschlüssige Verbindung ausgebildet sein. Im erfindungsgemäßen Verfahren werden der zweite und/oder dritte Schritt maschinell durchgeführt. Beispielsweise kann das Strömungsmodul mittels eines Roboters in die Trägerhülse eingeführt werden. Alternativ oder ergänzend kann die unlösbare Verbindung im dritten Schritt als Schweißverbindung von einem Roboter hergestellt werden. Das erfindungsgemäße Verfahren umfasst Schritte, die in einfacher und gleichzeitig präziser Weise maschinell umsetzbar sind. Dies ermöglicht eine beschleunigte Herstellung der Pneumatikmodule, die schnell in Gasanalysevorrichtungen montierbar und austauschbar sind. Die Pneumatikmodule sind separat auf ihre Wirkungsweise und Dichtigkeit prüfbar. Ein Überprüfen im eingebauten Zustand in der Gasanalysevorrichtung ist somit entbehrlich. Das erfindungsgemäße Verfahren ist ohne Weiteres an unterschiedlich dimensionierte Strömungsmodule und/oder Trägerhülsen anpassbar. Folglich erlaubt das erfindungsgemäße Verfahren eine prozesssichere, schnelle und damit kosteneffiziente Herstellung von Pneumatikmodulen.

Das mit dem erfindungsgemäßen Verfahren hergestellte Pneumatikmodul kann insbesondere gemäß einer der oben skizzierten Ausführungsformen ausgebildet sein, bei denen das Strömungsmodul im Bereich seines ersten Endes unlösbar mit der Trägerhülse verbunden ist. Die technischen Vorzüge der beanspruchten Pneumatikmodule übertragen sich damit analog auf das erfindungsgemäße Verfahren. Die in Verbindung mit den beanspruchten Pneumatikmodulen beschriebenen Merkmale gelten damit korrespondierend auch für das beanspruchte Verfahren.

Ferner wird die zugrundeliegende Aufgabenstellung durch ein erfindungsgemäßes Verfahren zum Simulieren eines Betriebsverhaltens eines Pneumatikmoduls in einer Gasanalysevorrichtung mit einem Computerprogrammprodukt ausgebildet ist und durch ein solches Computerprogrammprodukt selbst. Erfindungsgemäß ist das Pneumatikmodul gemäß einer der oben dargestellten Ausführungsformen ausgebildet.

Das Computerprogrammprodukt verfügt zur Simulation erfindungsgemäß über ein Physik-Modul, in dem das Pneumatikmodul zumindest teilweise abgebildet ist. Hierzu kann beispielsweise das Pneumatikmodul in seinem Aufbau und seiner Funktionsweise nachgebildet sein, beispielsweise als digitales Abbild, das zum Computerprogrammprodukt gehört. Alternativ oder ergänzend kann das Pneumatikmodul auch als Rechenmodell im Physik-Modul ausgebildet sein. Das Physik-Modul ist dazu ausgebildet, unter anderem das thermische oder aerodynamische Verhalten des Pneumatikmoduls unter einstellbaren Betriebsbedingungen nachzustellen. Das Pneumatikmodul weist eine Betriebstemperatur auf, die einer Temperatur der im Betrieb durchströmenden Fluidströmung entspricht. Zu den einstellbaren Betriebsbedingungen gehört erfindungsgemäß eine Umgebungstemperatur und optional eine Temperatur des zugeführten Fluids, eine Wärmeleitfähigkeit des Fluids und/oder der Wandung des Strömungsmoduls, eine Wärmeleitverhalten in der Trägerhülse, ein Zufuhrdruck, eine Zuflussgeschwindigkeit, und/oder eine Viskosität des Fluids gehören. Das Computerprogrammprodukt kann über eine Datenschnittstelle verfügten, über die entsprechende Daten über eine Benutzereingabe, eine Datenverbindung zu einem realen Pneumatikmodul bzw. Gasanalysevorrichtung und/oder andere simulationsgerichtete Computerprogramme vorgebbar sind. Ebenso kann das Computerprogrammprodukt über eine Datenschnittstelle zu einem Ausgeben von Simulationsresultaten an einen Benutzer und/oder andere simulationsgerichtete Computerprogrammprodukte verfügen. Mittels des Computerprogrammprodukts ist beispielsweise eine defektes Strömungsmodul, eine fehlerhafte Verbindung zwischen der Trägerhülse und dem Strömungsmodul und/oder eine Undichtigkeit an Zufuhrkanal und/oder Ablaufkanal des Pneumatikmoduls erkennbar. Insbesondere ist das Betriebsverhalten des Pneumatikmoduls, ausgedrückt durch Messwerte in Kanälen eines Leitungsblocks der Gasanalysevorrichtung, durch Abgleich mit dem simulierten Pneumatikmodul auf Plausibilität überprüfbar. Des Weiteren ist das Pneumatikmodul in einfacher Weise modellierbar, also mit einem Minimum an sogenannten CFD-Berechnungen im Betriebsverhalten nachrechenbar. Insbesondere kann das Durchströmungsverhalten im Strömungsmodul mit hinreichender Präzision durch algebraische Berechnung approximiert werden. Das erfindungsgemäße Computerprogrammprodukt erlaubt somit eine Modellierung des zugrundeliegenden Pneumatikmoduls bei einem reduzierten Bedarf an Rechenleistung. Hierdurch ist auch eine Vielzahl an derartigen Pneumatikmodulen, beispielsweise in einer Überwachungseinheit der Gasanalysevorrichtung nachbildbar. Somit ist insgesamt in einfacher Weise ein besonders realitätstreues Prozessabbild vom Betrieb der Gasanalysevorrichtung bereitstellbar. Das Computerprogrammprodukt kann als sogenannter Digitaler Zwilling ausgebildet sein, wie beispielsweise in der Druckschrift US 2017/286572 A1 beschrieben. Der Offenbarungsgehalt von US 2017/286572 A1 wird durch Verweisung in die vorliegende Anmeldung mit einbezogen. Das Computerprogrammprodukt kann monolithisch ausgebildet sein, also vollständig auf einer Hardwareplattform ausführbar. Alternativ kann das Computerprogrammprodukt modular ausgebildet sei und eine Mehrzahl an Teilprogrammen umfassen, die auf separaten Hardwareplattformen ausführbar sind und über eine kommunikative Datenverbindung zusammenwirken. Eine solche kommunikative Datenverbindung kann eine Netzwerkverbindung, eine Internetverbindung und/oder eine Mobilfunkverbindung sein. Ferner kann durch das erfindungsgemäße Computerprogrammprodukt ein Pneumatikmodul per Simulation erprobt und/oder optimiert werden.

Ebenso wird die Aufgabenstellung durch ein lösungsgemäßes Überwachungsverfahren für ein Pneumatikmodul gelöst. Das Überwachungsverfahren dient dazu, den Betrieb eines Pneumatikmoduls, das in einer Gasanalysevorrichtung eingesetzt wird, zu überwachen. Das Überwachungsverfahren umfasst einen ersten Schritt, in das Pneumatikmodul in der Gasanalysevorrichtung in einem aktiven Betriebszustand bereitgestellt wird. Ebenso wird im ersten Schritt zumindest ein Messwert bereitgestellt, der sich infolge einer strömungsmechanischen Einwirkung des Pneumatikmoduls auf eine Fluidströmung in der Gasanalysevorrichtung ergibt. Ebenso wird mindestens eine Betriebsgröße erfasst und bereitgestellt, durch die der vorliegende Betriebszustand der Gasanalysevorrichtung vorgegeben ist. Das Verfahren umfasst weiter einen zweiten Schritt, in dem die zumindest eine Betriebsgröße einem Computerprogrammprodukt als Eingabe bereitgestellt wird. Anhand der bereitgestellten Betriebsgröße wird durch das Computerprogrammprodukt im zweiten Schritt ein Soll-Messwert ermittelt, der mit dem im ersten Schritt bereitgestellten Messwert korrespondiert. Das Verfahren umfasst einen dritten Schritt, in dem der Soll-Messwert und der Messwert miteinander verglichen werden. Wenn eine Differenz zwischen dem Messwert und dem Soll-Messwert einen einstellbaren Schwellenwert betragsmäßig übersteigt, wird an einen Benutzer und/oder eine Steuereinheit der Gasanalysevorrichtung eine Warnung ausgegeben. Ergänzend kann in einem vierten Schritt anhand des Soll-Messwerts und des Messwerts über einen Erkennungsalgorithmus eine Ursache für die Differenz zwischen dem Messwert und dem Soll-Messwert identifiziert werden. Der Erkennungsalgorithmus kann beispielsweise als neuronales Netz ausgebildet sein. Lösungsgemäß ist das Computerprogrammprodukt, das im zweiten Schritt eingesetzt wird, nach einer der oben darstellten Ausführungsformen ausgebildet.

Die Erfindung wird im Folgenden anhand einzelner Ausführungsformen in Figuren näher erläutert. Die Figuren sind insoweit in gegenseitiger Ergänzung zu lesen, dass gleiche Bezugszeichen in unterschiedlichen Figuren die gleiche technische Bedeutung haben. Die Merkmale der einzelnen Ausführungsformen sind untereinander auch kombinierbar. Ferner sind die in den Figuren gezeigten Ausführungsformen mit den oben skizzierten Merkmalen kombinierbar. Es zeigen im Einzelnen:
- FIG 1: eine erste Ausführungsform des beanspruchten Pneumatikmoduls in einem Längsschnitt;
- FIG 2: eine zweite Ausführungsform des beanspruchten Pneumatikmoduls in einem Längsschnitt;
- FIG 3: eine dritte Ausführungsform des beanspruchten Pneumatikmoduls in einer Schrägansicht;
- FIG 4: eine Ausführungsform eines Teils einer beanspruchten Gasanalysevorrichtung in einer teiltransparenten Schrägansicht;
- FIG 5: schematisch einen Ablauf einer Ausführungsform des beanspruchten Verfahrens.

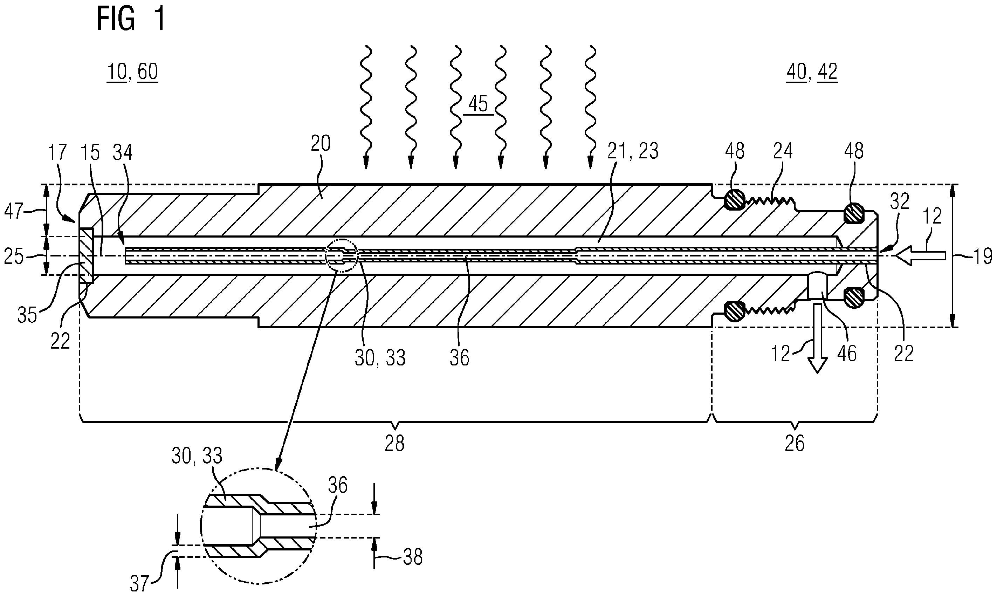

In FIG 1 ist ein Aufbau einer ersten Ausführungsform des beanspruchten Pneumatikmoduls 10 in einem Längsschnitt dargestellt. Das Pneumatikmodul 10 umfasst eine Trägerhülse 20, in der ein Innenraum 21 ausgebildet ist und in dem entlang einer Hauptachse 15 der Trägerhülse 20 ein Strömungsmodul 30 angeordnet ist. Die Trägerhülse 20 selbst ist einstückig ausgebildet. Das Strömungsmodul 30 ist als Quetschdrossel 33 ausgebildet und weist in einem mittleren Bereich eine Verengung 36 auf. Das Strömungsmodul 30 ist im Bereich eines ersten Endes 32 mit der Trägerhülse 20 über eine unlösbare Verbindung 22 verbunden, die als stoffschlüssige Verbindung ausgebildet ist. Das erste Ende 32 des Strömungsmoduls 30 ist derart in einem ersten Abschnitt 26 der Trägerhülse 20 angeordnet, dass eine Fluidströmung 12 in einem montierten Zustand des Pneumatikmoduls 10 eintreten kann. Die Fluidströmung 12 durchströmt das Strömungsmodul 30 und tritt an einem zweiten Ende 34 des Strömungsmoduls 30 in den Innenraum 21 der Trägerhülse 20 aus. Durch das Strömungsmodul 30 ist der Innenraum 21 der Trägerhülse 20 zumindest abschnittsweise als umlaufender lichter Zwischenraum 23 ausgebildet. Im ersten Abschnitt 26 der Trägerhülse 20 ist ein Austrittsöffnung 48 ausgebildet, die sich bezogen auf die Hauptachse 15 im Wesentlichen in einer Radialrichtung erstreckt. Die Fluidströmung 12 tritt über die Austrittsöffnung 46 in einem aktiven Betriebszustand des Pneumatikmoduls 10 aus. Weiter ist im ersten Abschnitt 26 der Trägerhülse 20 an einer Außenfläche der Trägerhülse 20 ein Gewinde 24 ausgebildet, das eine Montage des Pneumatikmoduls 10 in einen nicht näher gezeigten Leitungsblock 42 einer Gasanalysevorrichtung 40 erlaubt. Im ersten Abschnitt 26 der Trägerhülse 20 sind ferner Dichtungsmittel 48 angeordnet. Im Bereich des zweiten Endes 34 des Strömungsmoduls 30 ist an einem freien Ende 17 der Trägerhülse 20, und damit auch des Pneumatikmoduls 10, ein Deckel 35 angeordnet. Der Deckel 35 ist ebenfalls durch eine unlösbare Verbindung 22 mit der Trägerhülse 20 verbunden. Durch den Deckel 35 ist die Trägerhülse 20 verschlossen ausgebildet.

Durch die Trägerhülse 20 wird für das Strömungsmodul 30 eine mechanische Abschirmung gegenüber der Umgebung gewährleistet. Ebenso wird das Strömungsmodul 30 gegenüber einem Wärmeeintrag 45 aus der Umgebung abgeschirmt. Zur Gewährleistung der mechanischen und thermischen Abschirmung des Strömungsmoduls 30 weist die Trägerhülse 20 eine hinreichende Hülsenwandstärke 47 auf und das Strömungsmodul 30 eine Wandstärke 37. Weiter weist die Trägerhülse einen maximalen Außendurchmesser 19 auf. Dadurch wird eine hinreichende mechanische Stabilität der Trägerhülse 20 gewährleistet, die auch eine sichere Handhabung des Pneumatikmoduls 10 erlaubt, jedoch gleichzeitig kompakt ist. Dementsprechend ist das Strömungsmodul 30 filigran ausgebildet und weist eine Wandstärke 37 von bis zu 5 mm und/oder einen minimalen Innendurchmesser 38 von 1 µm bis zu 2,0 mm auf. auf. Weiter ist die unlösbare Verbindung 22 zwischen der Trägerhülse 20 und dem Strömungsmodul 30 kompakt ausgebildet. Dadurch wirkt die Trägerhülse 20 teilweise als thermische Senke und die Auswirkung eines Wärmeeintrags 45 aus der Umgebung auf das Strömungsmodul 30 wird reduziert.

Sowohl die Trägerhülse 20 und das Strömungsmodul 30 sind aus einem metallischen Werkstoff hergestellt. Die jeweiligen Werkstoffe sind dahingehend gewählt, dass diese in puncto Schweißbarkeit eine vorteilhafte Materialpaarung bilden. Die unlösbare Verbindung 22 ist maschinell durch Laserschweißen hergestellt. Dadurch wird bei der Herstellung des Pneumatikmoduls 10 ein Wärmeeintrag in das Strömungsmodul 30 minimiert und einem Verziehen des Strömungsmoduls 30 vorgebeugt. Das Pneumatikmodul 10 ist folglich mit einer gesteigerten Prozesssicherheit präzise herstellbar. Das Pneumatikmodul 10 ist durch ein Computerprogrammprodukt 60 abgebildet, das als sogenannter Digitaler Zwilling ausgebildet ist. Das Computerprogrammprodukt 60 ist dazu ausgebildet, das Betriebsverhalten des Pneumatikmoduls 10 zu simulieren. Insbesondere ist ein Verhalten der Fluidströmung 12 bei einem Durchströmen des Pneumatikmoduls 10 dadurch simulierbar.

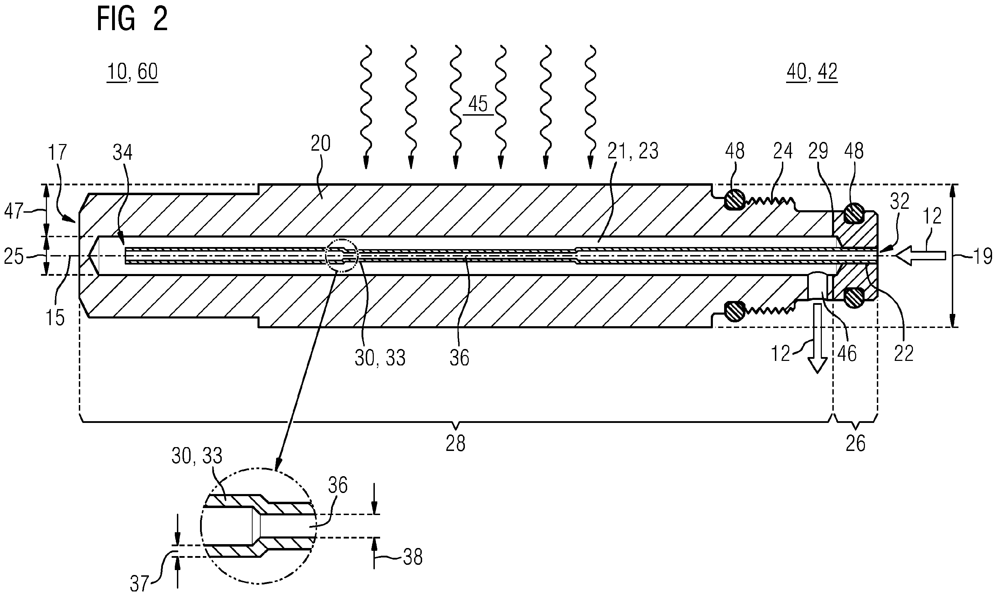

Eine zweite Ausführungsform des beanspruchten Pneumatikmoduls 10 ist in FIG 2 in einem Längsschnitt parallel zu dessen Hauptachse 15 dargestellt. Das Pneumatikmodul 10 umfasst eine Trägerhülse 20, in der ein Innenraum 21 ausgebildet ist und in dem entlang einer Hauptachse 15 der Trägerhülse 20 ein Strömungsmodul 30 angeordnet ist. Die Trägerhülse 20 umfasst einen ersten Abschnitt 26 und einen daran angrenzenden zweiten Abschnitt 28. Der erste und zweite Abschnitt 26, 28 sind an einer Fügestelle 29 miteinander verbunden und jeweils aus unterschiedlichen metallischen Werkstoffen hergestellt. Die Fügestelle 29 ist als stoffschlüssige Verbindung ausgebildet. Das Strömungsmodul 30 ist als Quetschdrossel 33 ausgebildet und weist in einem mittleren Bereich eine Verengung 36 auf. Das Strömungsmodul 30 ist im Bereich eines ersten Endes 32 mit dem ersten Abschnitt 26 der Trägerhülse 20 über eine unlösbare Verbindung 22 verbunden, die als stoffschlüssige Verbindung ausgebildet ist. Das erste Ende 32 des Strömungsmoduls 30 ist derart im ersten Abschnitt 26 der Trägerhülse 20 angeordnet, dass eine Fluidströmung 12 in einem montierten Zustand des Pneumatikmoduls 10 eintreten kann. Das Strömungsmodul 30 ist bei der Herstellung mit dem ersten Abschnitt 26 der Trägerhülse 28 verbindbar. Der zweite Abschnitt 28 der Trägerhülse 30 ist anschließend mit dem ersten Abschnitt 26 verbindbar. Dadurch wird die Handhabung des Strömungsmoduls 30 bei der Herstellung weiter vereinfacht.

Die Fluidströmung 12 durchströmt das Strömungsmodul 30 und tritt an einem zweiten Ende 34 des Strömungsmoduls 30 in den Innenraum 21 der Trägerhülse 20 aus. Durch das Strömungsmodul 30 ist der Innenraum 21 der Trägerhülse 20 zumindest abschnittsweise als umlaufender lichter Zwischenraum 23 ausgebildet. Im ersten Abschnitt 26 der Trägerhülse 20 ist ein Austrittsöffnung 48 ausgebildet, die sich bezogen auf die Hauptachse 15 im Wesentlichen in einer Radialrichtung erstreckt. Die Fluidströmung 12 tritt über die Austrittsöffnung 46 in einem aktiven Betriebszustand des Pneumatikmoduls 10 aus. Weiter ist im ersten Abschnitt 26 der Trägerhülse 20 an einer Außenfläche der Trägerhülse 20 ein Gewinde 24 ausgebildet, das eine Montage des Pneumatikmoduls 10 in einen nicht näher gezeigten Leitungsblock 42 einer Gasanalysevorrichtung 40 erlaubt. Im ersten Abschnitt 26 der Trägerhülse 20 sind ferner Dichtungsmittel 48 angeordnet. Im Bereich des zweiten Endes 34 des Strömungsmoduls 30 ist die Trägerhülse 20 an einem freien Ende 17 verschlossen ausgebildet. Gegenüber der in FIG 1 gezeigten ersten Ausführungsform ist deren Deckel 35 in der Ausführungsform nach FIG 2 eingespart.

Durch die Trägerhülse 20 wird für das Strömungsmodul 30 eine mechanische Abschirmung gegenüber der Umgebung gewährleistet. Ebenso wird das Strömungsmodul 30 gegenüber einem Wärmeeintrag 45 aus der Umgebung abgeschirmt. Zur Gewährleistung der mechanischen und thermischen Abschirmung des Strömungsmoduls 30 weist die Trägerhülse 20 in ihrem zweiten Abschnitt 28 eine hinreichende Hülsenwandstärke 47 auf. Weiter weist die Trägerhülse 20 in ihrem zweiten Abschnitt 28 einen maximalen Außendurchmesser 19 auf. Dadurch wird eine hinreichende mechanische Stabilität der Trägerhülse 20 gewährleistet, die auch eine sichere Handhabung des Pneumatikmoduls 10 erlaubt, jedoch gleichzeitig kompakt ist. Dementsprechend ist das Strömungsmodul 30 filigran ausgebildet und weist eine Wandstärke 37 von bis zu 5 mm und/oder einen minimalen Innendurchmesser 38 von 1 µm bis zu 2,0 mm auf. auf. Weiter ist die unlösbare Verbindung 22 zwischen der Trägerhülse 20, also deren erstem Abschnitt 26, und dem Strömungsmodul 30 kompakt ausgebildet. Dadurch wirkt die Trägerhülse 20 teilweise als thermische Senke und die Auswirkung eines Wärmeeintrags 45 aus der Umgebung auf das Strömungsmodul 30 wird reduziert.

Sowohl die Trägerhülse 20 und das Strömungsmodul 30 sind aus einem metallischen Werkstoff hergestellt. Die jeweiligen Werkstoffe sind dahingehend gewählt, dass diese in puncto Schweißbarkeit eine vorteilhafte Materialpaarung bilden. Die unlösbare Verbindung 22 ist maschinell durch Laserschweißen hergestellt. Dadurch wird bei der Herstellung des Pneumatikmoduls 10 ein Wärmeeintrag in das Strömungsmodul 30 minimiert und einem Verziehen des Strömungsmoduls 30 vorgebeugt. Das Pneumatikmodul 10 ist folglich mit einer gesteigerten Prozesssicherheit präzise herstellbar. Das Pneumatikmodul 10 ist durch ein Computerprogrammprodukt 60 abgebildet, das als sogenannter Digitaler Zwilling ausgebildet ist. Das Computerprogrammprodukt 60 ist dazu ausgebildet, das Betriebsverhalten des Pneumatikmoduls 10 zu simulieren. Insbesondere ist ein Verhalten der Fluidströmung 12 bei einem Durchströmen des Pneumatikmoduls 10 dadurch simulierbar.

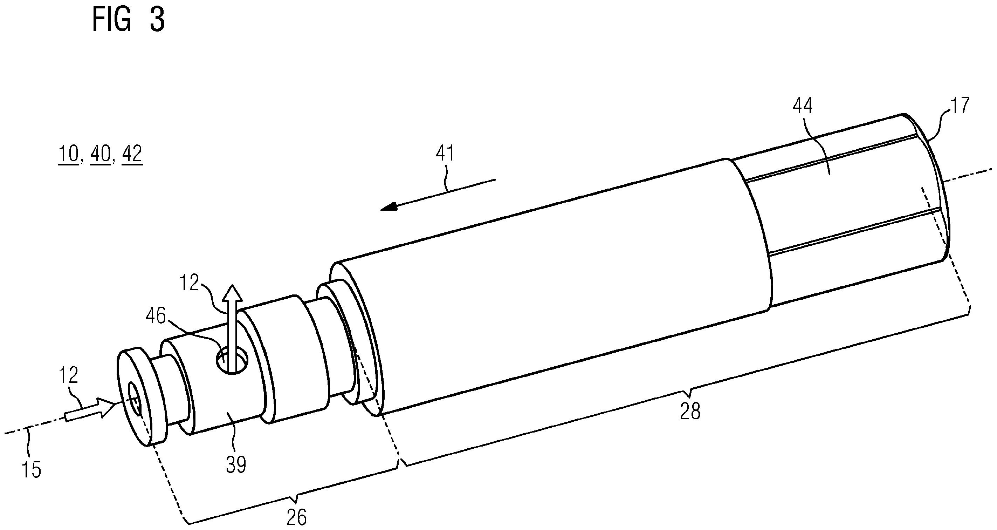

Eine dritte Ausführungsform des beanspruchten Pneumatikmoduls 10 ist in FIG 3 in einer Schrägansicht dargestellt. Das Pneumatikmodul 10 umfasste eine Trägerhülse 20, die sich im Wesentlichen entlang einer Hauptachse 15 erstreckt. Die Trägerhülse 10 weist einen ersten Abschnitt 26 auf, der dazu ausgebildet ist, eine Fluidströmung 12 in das Pneumatikmodul 10 einzuleiten. Der erste Abschnitt 26 ist mit Haltevorsprüngen und Absätzen versehen, die eine Montage in einem nicht näher gezeigten Leitungsblock 42 einer Gasanalysevorrichtung 40 erlaubt. Zur Befestigung des Pneumatikmoduls 10 in der Gasanalysevorrichtung 40 wird dieses in eine Montagerichtung 41 in den Leitungsblock 42 eingeführt. Durch die Haltevorsprünge und Absätze ist der erste Abschnitt 26 der Trägerhülse 20 als Druckluftanschluss 39 ausgebildet. Im ersten Abschnitt 26 ist ferner eine Austrittsöffnung 46 ausgebildet, durch die im Betrieb des Pneumatikmoduls 10 eine Fluidströmung 12 austritt. In einem zweiten Abschnitt 28 der Trägerhülse 20, der an den ersten Abschnitt 26 angrenzt, ist im Bereich eines freien Endes 17 ein Werkzeugansatz 44 ausgebildet. Der Werkzeugansatz 44 ist kantig, insbesondere sechskantig, ausgebildet, so dass das Pneumatikmodul 10 zu einer Montage oder Demontage am Werkzeugansatz 44 mit einem Werkzeug greifbar ist. In der Trägerhülse 20 ist ein nicht näher gezeigtes Strömungsmodul 30 aufgenommen, das durch die eintretende Fluidströmung 12 durchströmt wird. Die an der Austrittsöffnung 46 austretende Fluidströmung 12 weist gegenüber der eintretenden Fluidströmung 12 einen Totaldruckverlust auf. Das Pneumatikmodul 10 kann innen beispielsweise gemäß FIG 1 oder FIG 2 ausgebildet sein. Das Pneumatikmodul 10 ist ebenso durch ein nicht näher abgebildetes Computerprogrammprodukt 60 in seinem Betriebsverhalten simulierbar. Das Computerprogrammprodukt 60 ist als sogenannter Digitaler Zwilling ausgebildet.

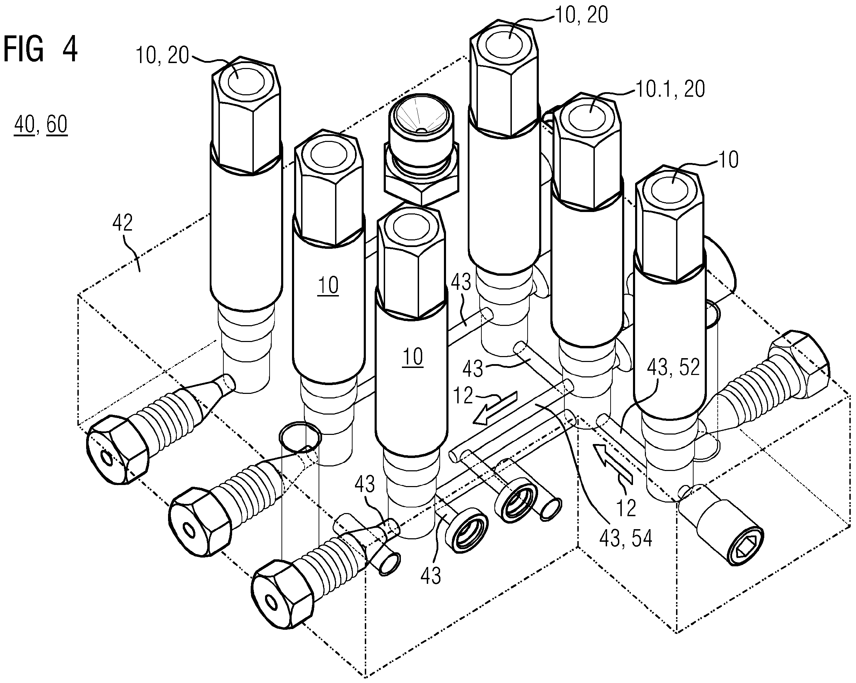

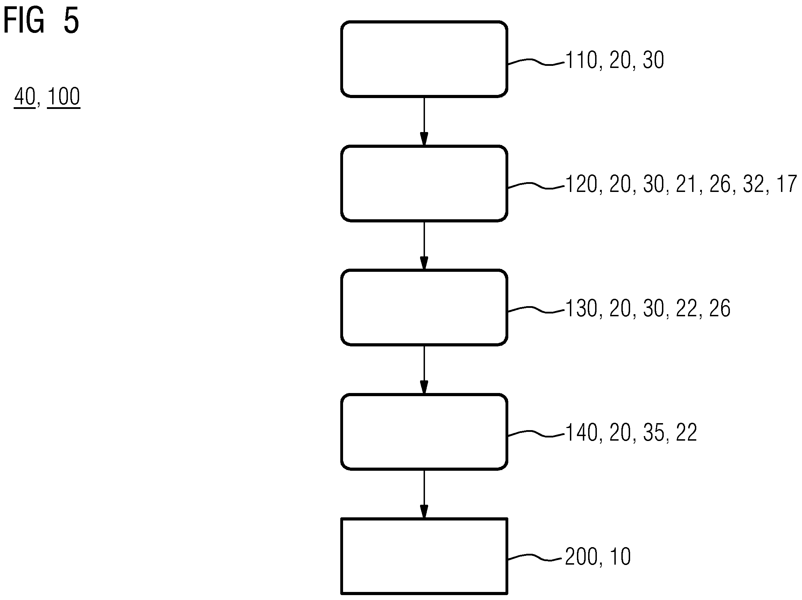

FIG 4 zeigt schematisch einen Teil einer Ausführungsform einer beanspruchten Gasanalysevorrichtung 40. Zur Gasanalysevorrichtung 40 gehört ein Leitungsblock 42, in dem eine Mehrzahl an Kanälen 43 ausgebildet ist. Die Kanäle 42 sind Ausnehmungen innerhalb des Leitungsblocks 42. Zur besseren Übersicht ist der Leitungsblock 42 in FIG 3 transparent dargestellt. Im Leitungsblock 42 ist weiter eine Mehrzahl an Pneumatikmodulen 10 lösbar, also zerstörungsfrei lösbar, aufgenommen. Ein erstes Pneumatikmodul 10.1 ist mit einem Kanal 43 verbunden, der als Zufuhrkanal 52 ausgebildet ist, und durch den eine Fluidströmung 12 in das erste Pneumatikmodul 10.1 geleitet wird. Nach einem Durchströmen des ersten Pneumatikmoduls 10.1 tritt die Fluidströmung 12 über einen Kanal 43 aus, der als Ablaufkanal 54 dient. Dementsprechend wird der Zufuhrkanal 52 über das erste Pneumatikmodul 10.1 mit dem Ablaufkanal 54 verbunden. Die Kanäle 43 sind über die weiteren Pneumatikmodule 10 gleichermaßen verbunden. Die Kanäle 43 sind dadurch, dass sie als Ausnehmungen im Leitungsblock 42 ausgebildet sind, sind diese gegen Einwirkungen aus der Umgebung robust, insbesondere gegen mechanische Einwirkungen. Gleichermaßen sind die Pneumatikmodule 10, 10.1 robust gegen mechanische und thermische Einwirkungen aus der Umgebung. Die Pneumatikmodule 10, 10.1 können nach einer der in FIG 1, FIG 2 oder FIG 3 gezeigten Ausführungsformen ausgebildet sein und weisen entsprechend reduzierte Abmessungen auf. Die Kanäle 43 weisen Durchmesser auf, die an die Abmessungen der Pneumatikmodule 10, 10.1 angepasst sind. Dementsprechend ist der Leitungsblock 42 kompakt ausgebildet. Dies ermöglicht eine verstärkte Miniaturisierung der zugehörigen Gasanalysevorrichtung 40 bei gleichzeitig gesteigerter Robustheit. Ferner weisen die Pneumatikmodule 10, 10.1 Trägerhülsen 20 auf, die als Gleichteile ausgebildet sind. Die Pneumatikmodule 10, 10.1 sind damit gegenseitig austauschbar. Die Pneumatikmodule 10 sind in einfacher Weise austauschbar, so dass eine Reparatur der Gasanalysevorrichtung 40 beschleunigt wird. Ferner ist der Leitungsblock 42 als Gleichteil für unterschiedliche Bautypen an Gasanalysevorrichtungen 40 einsetzbar. Der Leitungsblock 42 ist durch ein Versehen mit unterschiedlichen Pneumatikmodulen 10, 10.1 in seiner strömungsmechanischen Charakteristik einstellbar, wobei sich die Pneumatikmodule 10, 10.2 durch ihre jeweiligen Strömungsmodule 30 unterscheiden. Der in FIG 5 gezeigte Teil der Gasanalysevorrichtung 40 verwirklicht somit ein Baukastenkonzept für unterschiedliche Bautypen von Gasanalysevorrichtungen 40. Ferner ist das Betriebsverhalten von zumindest einem Pneumatikmodul 10, 10.1 während eines Betriebs der Gasanalysevorrichtung 40 mittels eines Computerprogrammprodukts 60 simulierbar, das als Digitaler Zwilling des jeweiligen Pneumatikmoduls 10, 10.1 ausgebildet ist.

Ein Ablauf einer Ausführungsform des beanspruchten Verfahrens 100 ist in FIG 5 schematisch dargestellt. Das Verfahren 100 dient zu einem Herstellen eines Pneumatikmoduls 10, das zur Verwendung in einer nicht näher gezeigten Gasanalysevorrichtung 40 ausgebildet ist. Das Verfahren 100 umfasst einen ersten Schritt 110, in dem eine Trägerhülse 20 und ein Strömungsmodul 30 bereitgestellt werden, die als Werkstücke für das Pneumatikmodul 10 dienen. Daran schließt sich ein zweiter Schritt 120 an, in dem das Strömungsmodul 30 in einen Innenraum 21 der Trägerhülse 20 eingeführt wird. Das Einführen erfolgt durch eine Ausnehmung an einem freien Ende 17 der trägerhülse 20. Das Strömungsmodul 30 liegt im Bereich eines ersten Endes 32 in einem ersten Abschnitt 26 der Trägerhülse 20 an und ist dazu ausgerichtet, eine Fluidströmung 12 in den Innenraum 21 der Trägerhülse 20 zu leiten. Der erste Abschnitt 26 der Trägerhülse 20 liegt an einem dem freien Ende 17 abgewandten Ende der Trägerhülse 20. Insgesamt erreicht das Strömungsmodul 20 im zweiten Schritt 120 eine vorgesehene Montageposition. Der zweite Schritt 120 wird maschinell, insbesondere mittels eines Roboters, durchgeführt.

Weiter umfasst das Verfahren 100 einen darauffolgenden dritten Schritt 130, in dem zwischen der Trägerhülse 20 und dem Strömungsmodul 30 eine unlösbare Verbindung 22 hergestellt wird. Die unlösbare Verbindung 22 ist als stoffschlüssige Verbindung ausgebildet und ist höchstens noch zerstörungsbehaftet wieder lösbar. Die unlösbare Verbindung 22 wird im Bereich des ersten Endes 32 des Strömungsmoduls 30 ausgebildet. Auch der dritte Schritt 130 wird maschinell durchgeführt, insbesondere mittels eines Roboters. An den dritten Schritt 130 schließt sich ein vierter Schritt 140 an, in dem ein Deckel 35 bereitgestellt wird und am freien Ende 17 der Trägerhülse 20 positioniert. Die Ausnehmung am freien Ende 17 der Trägerhülse 20 wird durch den Deckel 35 verschlossen. Weiter wird im vierten Schritt 140 eine unlösbare Verbindung 22 zwischen dem Deckel 35 und der Trägerhülse 20 hergestellt. Nach dem vierten Schritt 140 erreicht das Verfahren 100 einen Endzustand 200, in dem das so hergestellte Pneumatikmodul 10 vorliegt. Das mittels des Verfahrens 100 hergestellte 10 Pneumatikmodul 10 wird in einem nicht näher gezeigten Computerprogrammprodukt 60 dargestellt, das dazu geeignet ist dessen Betriebsverhalten zu simulieren.

## Patentansprüche

1. Gasanalysevorrichtung (40), umfassend einen Leitungsblock (42) mit einer Mehrzahl an Kanälen (43), wobei zumindest zwei Kanäle (43) über ein austauschbares Pneumatikmodul (10) miteinander verbunden sind, wobei das Pneumatikmodul (10) zum Einstellen einer Fluidströmung (12) in einer Gasanalysevorrichtung (40) ausgebildet ist und eine Trägerhülse (20) und ein Strömungsmodul (30) umfasst, das in der Trägerhülse (20) aufgenommen ist, wobei das Strömungsmodul (30) an einem ersten Ende (32) zu einer Steigerung einer Messgenauigkeit der Gasanalysevorrichtung (40) mit der Trägerhülse (20) verbunden ist, **dadurch gekennzeichnet, dass** die Trägerhülse (20) im Bereich des ersten Endes (32) des Strömungsmoduls (30) zu einer Montage an der Gasanalysevorrichtung (40) ausgebildet ist und im Bereich eines zweiten Endes (34) des Strömungsmoduls (34) verschlossen ausgebildet ist.

2. Gasanalysevorrichtung (40) nach Anspruch 1, **dadurch gekennzeichnet, dass** das Strömungsmodul (30) im Bereich seines ersten Endes (32) lösbar mit der Trägerhülse (20) verbunden ist.

3. Gasanalysevorrichtung (40) nach Anspruch 1, **dadurch gekennzeichnet, dass** das Strömungsmodul (30) im Bereich seines ersten Endes (32) unlösbar mit der Trägerhülse (20) verbunden ist.

4. Gasanalysevorrichtung (40) nach einem der Ansprüche 1 bis 3, **dadurch gekennzeichnet, dass** ein zweites Ende (34) des Strömungsmoduls (30) in einen Innenraum (21) der Trägerhülse (20) mündet.

5. Gasanalysevorrichtung (40) nach einem der Ansprüche 1 bis 4, **dadurch gekennzeichnet, dass** die Trägerhülse (20) einstückig oder mehrstückig ausgebildet ist.

6. Gasanalysevorrichtung (40) nach einem der Ansprüche 1 bis 5, **dadurch gekennzeichnet, dass** die Trägerhülse (20) im Bereich des zweiten Endes (34) des Strömungsmoduls (30) durch einen unlösbar befestigten Deckel (35) verschlossen ausgebildet ist.

7. Gasanalysevorrichtung (40) nach einem der Ansprüche 1 bis 6, **dadurch gekennzeichnet, dass** das Strömungsmodul (30) und/oder die Trägerhülse (20) zumindest teilweise aus einem metallischen Werkstoff hergestellt sind.

8. Gasanalysevorrichtung (40) nach einem der Ansprüche 1 bis 7, **dadurch gekennzeichnet, dass** das Strömungsmodul (30) einen minimalen Innendurchmesser (38) von 1 µ bis zu 2,0 mm aufweist und/oder eine Wandstärke (37) von bis zu 5,0 mm.

9. Gasanalysevorrichtung (40) nach einem der Ansprüche 1 bis 8, **dadurch gekennzeichnet, dass** das Strömungsmodul (30) als Quetschdrossel (33) ausgebildet ist.

10. Gasanalysevorrichtung (40) nach einem der Ansprüche 1 bis 9, **dadurch gekennzeichnet, dass** die Gasanalysevorrichtung (40) als Gasanalysator oder als Gaschromatograph ausgebildet ist.

11. Verfahren (100) zum Herstellen eines Pneumatikmoduls (10) für eine Gasanalysevorrichtung (40), umfassend die Schritte:
a) Bereitstellen einer Trägerhülse (20) und eines Strömungsmoduls (30);
b) Einführen des Strömungsmoduls (30) in einen Innenraum (21) der Trägerhülse (20);
c) Herstellen einer unlösbaren Verbindung (22) zwischen der Trägerhülse (20) und dem Strömungsmodul (30) im Bereich eines ersten Endes (32) des Strömungsmoduls (30);
wobei Schritt b) und/oder c) maschinell durchgeführt wird, **dadurch gekennzeichnet, dass** die Gasanalysevorrichtung (40) und das Pneumatikmodul (30) nach Anspruch 3 ausgebildet sind.

12. Verfahren (100) nach Anspruch 11, **dadurch gekennzeichnet, dass** die Gasanalysevorrichtung (40) und das Pneumatikmodul (10) weiter nach einem der Ansprüche 4 bis 10 ausgebildet sind und die Ansprüche 4 bis 10 nicht von Anspruch 2 abhängen.

13. Verfahren zum Simulieren eines Betriebsverhaltens eines Pneumatikmoduls (10) mittels eines Computerprogrammprodukt (60), dem eine Fluidströmung (12) zugeführt wird, wobei das Computerprogrammprodukt (60) eine Abbildung des Pneumatikmoduls (10) umfasst, **dadurch gekennzeichnet, dass** das Pneumatikmodul (10) als Pneumatikmodul (10) in einer Gasanalysevorrichtung (40) nach einem der Ansprüche 1 bis 10 ausgebildet ist, und das Computerprogrammprodukt (60) zur Simulation über ein Physik-Modul verfügt, in dem das Pneumatikmodul (10) zumindest teilweise abgebildet wird, und das das thermische Verhalten des Pneumatikmoduls (10) unter einstellbaren Betriebsbedingungen nachstellt, wobei das Pneumatikmodul (10) eine Betriebstemperatur aufweist, die einer Temperatur der im Betrieb durchströmenden Fluidströmung (12) entspricht, und wobei eine Umgebungstemperatur zu den einstellbaren Betriebsbedingungen gehört, und das Strömungsmodul (30) von der Trägerhülse (20) gegen thermische Einwirkungen aus einer Umgebung abgeschirmt wird.

14. Computerprogrammprodukt (60) zum Simulieren eines Betriebsverhaltens eines Pneumatikmoduls (10), dem eine Fluidströmung (12) zugeführt wird, wobei das Computerprogrammprodukt (60) eine Abbildung des Pneumatikmoduls (10) umfasst, **dadurch gekennzeichnet, dass** das Computerprogrammprodukt (60) dazu ausgebildet ist, ein Verfahren nach Anspruch 13 durchzuführen.

## Claims

1. Gas analysis device (40), comprising a conduction block (42) which has a plurality of channels (43), wherein at least two channels (43) are connected together via an exchangeable pneumatic module (10), wherein the pneumatic module (10) is designed for adjusting a fluid flow (12) in a gas analysis device (40) and comprises a support sleeve (20) and a flow module (30) which is contained in the support sleeve (20), wherein the flow module (30) is connected at a first end (32) thereof to the support sleeve (20) in order to achieve greater measuring accuracy of the gas analysis device (40), **characterised in that** the support sleeve (20) is designed in the region of the first end (32) of the flow module (30) for assembly on the gas analysis device (40) and is designed to be closed in the region of a second end (34) of the flow module (34) .

2. Gas analysis device (40) according to claim 1, **characterised in that** the flow module (30) in the region of its first end (32) is connected in a detachable manner to the support sleeve (20).

3. Gas analysis device (40) according to claim 1, **characterised in that** the flow module (30) in the region of its first end (32) is connected in a non-detachable manner to the support sleeve (20).

4. Gas analysis device (40) according to one of claims 1 to 3, **characterised in that** a second end (34) of the flow module (30) opens into an interior chamber (21) of the support sleeve (20) .

5. Gas analysis device (40) according to one of claims 1 to 4, **characterised in that** the support sleeve (20) is designed as a single part or multiple parts.

6. Gas analysis device (40) according to one of claims 1 to 5, **characterised in that** the support sleeve (20) is designed to be closed in the region of a second end (34) of the flow module (34) by a cover (35) fixed in a non-detachable manner.

7. Gas analysis device (40) according to one of claims 1 to 6, **characterised in that** the flow module (30) and/or the support sleeve (20) is produced at least partially from a metallic material.

8. Gas analysis device (40) according to one of claims 1 to 7, **characterised in that** the flow module (30) has a minimum inner diameter (38) of 1 µ to 2.0 mm and/or a wall thickness (37) of up to 5.0 mm.

9. Gas analysis device (40) according to one of claims 1 to 8, **characterised in that** the flow module (30) is designed as a pinch throttle (33).

10. Gas analysis device (40) according to one of claims 1 to 9, **characterised in that** the gas analysis device (40) is designed as a gas analyser or as a gas chromatograph.

11. Method (100) for producing a pneumatic module (10) for a gas analysis device (40), comprising the steps:
a) providing a support sleeve (20) and a flow module (30);
b) inserting the flow module (30) into an interior chamber (21) of the support sleeve (20);
c) producing a non-detachable connection (22) between the support sleeve (20) and the flow module (30) in the region of a first end (32) of the flow module (30);
wherein step b) and/or c) is performed automatically, **characterised in that** the gas analysis device (40) and the pneumatic module (30) are designed according to claim 3.

12. Method (100) according to claim 11, **characterised in that** the gas analysis device (40) and the pneumatic module (30) are further designed according to one of claims 4 to 10 and claims 4 to 10 do not depend on claim 2.

13. Method for the simulation of operating characteristics of a pneumatic module (10) by means of a computer program product (60), into which a fluid flow (12) is fed, wherein the computer program product (60) comprises a representation of the pneumatic module (10), **characterised in that** the pneumatic module (10) is designed as a pneumatic module (10) in a gas analysis device (40) according to one of claims 1 to 10 and the computer program product (60) has a physics module for simulation, in which the pneumatic module (10) is at least partially represented and which portrays the thermal characteristics of the pneumatic module (10) under operating conditions that can be adjusted, wherein the pneumatic module (10) has an operating temperature which corresponds to a temperature of the fluid flow (12) flowing during operation, and wherein an ambient temperature forms part of the adjustable operating conditions and the flow module (30) is shielded against thermal effects from an environment by the support sleeve (20).

14. Computer program product (60) for the simulation of operating characteristics of a pneumatic module (10), into which a fluid flow (12) is fed, wherein the computer program product (60) comprises a representation of the pneumatic module (10), **characterised in that** the computer program product (60) is designed to carry out a method according to claim 13.

## Revendications

1. Dispositif (40) d'analyse de gaz, comprenant un bloc (42) de conduite ayant une pluralité de conduits (43), dans lequel au moins deux conduits (43) communiquent entre eux par un module (10) pneumatique remplaçable, dans lequel le module (10) pneumatique est constitué pour l'établissement d'un courant (12) de fluide dans un dispositif (40) d'analyse de gaz et comprend un manchon (20) support et un module (30) d'écoulement, qui est reçu dans le manchon (20) support, dans lequel le module (30) d'écoulement est, à une première extrémité (32), assemblé au manchon (20) support pour augmenter la précision de mesure du dispositif (40) d'analyse de gaz, **caractérisé en ce que** le manchon (20) support est, dans la partie de la première extrémité (32) du module (30) d'écoulement, constitué pour un montage sur le dispositif (40) d'analyse de gaz et est constitué fermé dans la partie de la deuxième extrémité (34) du module (34) d'écoulement.

2. Dispositif (40) d'analyse de gaz suivant la revendication 1, **caractérisé en ce que** le module (30) d'écoulement est, dans la partie de sa première extrémité (32), assemblé de manière détachable au manchon (20) support.

3. Dispositif (40) d'analyse de gaz suivant la revendication 1, **caractérisé en ce que** le module (30) d'écoulement est, dans la partie de sa première extrémité (32), assemblé de manière indissoluble au manchon (20) support.

4. Dispositif (40) d'analyse de gaz suivant l'une des revendications 1 à 3, **caractérisé en ce que** la deuxième extrémité (34) du module (30) d'écoulement débouche dans l'espace (21) intérieur du manchon (20) support.

5. Dispositif (40) d'analyse de gaz suivant l'une des revendications 1 à 4, **caractérisé en ce que** le manchon (20) support est en une seule pièce ou en plusieurs pièces.

6. Dispositif (40) d'analyse de gaz suivant l'une des revendications 1 à 5, **caractérisé en ce que** le manchon (20) support est, dans la partie de la deuxième extrémité (34) du module (30) d'écoulement constitué fermé par un couvercle (35) fermé de manière indétachable.

7. Dispositif (40) d'analyse de gaz suivant l'une des revendications 1 à 6, **caractérisé en ce que** le module (30) d'écoulement et/ou le manchon (20) support sont fabriqués au moins en partie en un matériau métallique.

8. Dispositif (40) d'analyse de gaz suivant l'une des revendications 1 à 7, **caractérisé en ce que** le module (30) d'écoulement a un diamètre (38) intérieur minimum de 1 µ à 2,0 mm et/ou une épaisseur (37) allant jusqu'à 5,0 mm.

9. Dispositif (40) d'analyse de gaz suivant l'une des revendications 1 à 8, **caractérisé en ce que** le module (30) d'écoulement est constitué sous la forme d'une bobine (33) de compression.

10. Dispositif (40) d'analyse de gaz suivant l'une des revendications 1 à 9, **caractérisé en ce que** le dispositif (40) d'analyse de gaz est constitué sous la forme d'un analyseur de gaz ou d'un chromatographe en phase gazeuse.

11. Procédé (100) de fabrication d'un module (10) pneumatique pour un dispositif (40) d'analyse de gaz, comprenant les stades :
a.) on se procure un manchon (20) support et un module (30) d'écoulement ;
b.) on introduit le module (30) d'écoulement dans un espace (21) intérieur du manchon (20) support ;
c.) on produit un assemblage (22) indissoluble entre le manchon (20) support et le module (30) d'écoulement dans la partie d'une première extrémité (32) du module (30) d'écoulement ;
dans lequel on effectue à la machine le stade b) ou c), **caractérisé en ce que** le dispositif (40) d'analyse de gaz et le module (30) pneumatique sont constitués suivant la revendication 3.

12. Procédé (100) suivant la revendication 11, **caractérisé en ce que** le dispositif (40) d'analyse de gaz et le module (10) pneumatique sont constitués en outre suivant l'une des revendications 4 à 10 et les revendications 4 à 10 ne dépendent pas de la revendication 2.

13. Procédé de simulation d'un comportement de fonctionnement d'un module (10) pneumatique au moyen d'un produit (60) de programme d'ordinateur, auquel est envoyé un écoulement (12) de fluide, dans lequel le produit (60) de programme d'ordinateur comprend une représentation du module (10) pneumatique, **caractérisé en ce que** le module (10) pneumatique est constitué sous la forme d'un module (10) pneumatique dans un dispositif (40) d'analyse de gaz suivant l'une des revendications 1 à 10, et le produit (60) de programme d'ordinateur dispose, pour la simulation, d'un module physique, dans lequel le module (10) pneumatique peut être représenté au moins en partie, et qui règle le comportement thermique du module (10) pneumatique dans des conditions de fonctionnement réglables, dans lequel le module (10) pneumatique a une température de fonctionnement, qui correspond à une température du courant (12) de fluide passant en fonctionnement, et dans lequel une température ambiante appartient aux conditions de fonctionnement réglables, et le module (30) d'écoulement est protégé par le manchon (20) support des effets thermiques d'un environnement.

14. Produit (60) de programme d'ordinateur pour la simulation d'un comportement de fonctionnement d'un module (10) pneumatique, auquel est envoyé un écoulement (12) de fluide, dans lequel le produit (60) de programme d'ordinateur comprend une représentation du module (10) pneumatique, **caractérisé en ce que** le produit (60) de programme d'ordinateur est constitué pour exécuter un procédé suivant la revendication 13.
